# EUROPEAN PATENT APPLICATION

(11) **EP 4 708 314 A1**
(43) Date of publication of application: **11.03.2026**
(21) Application number: 24815806.5
(22) Date of filing: 24.05.2024
(51) Int. Cl.: G16H 20/70, G16H 10/60, G16H 10/20, A61B 5/00, G09B 7/02

(54) **APPARATUS AND METHOD FOR PROVIDING COGNITIVE TRAINING BASED ON GAME CONTENT FOR ALTERNATELY PROVIDING OVERALL BRAIN STIMULATION AND BRAIN STIMULATION OF SPECIFIC PART**

(30) Priority: 26.05.2023 KR 20230068465
(71) Applicant: NUNAPS INC., Seoul 05505 (KR); The Asan Foundation, Seoul 05505 (KR); University Of Ulsan Foundation For Industry Cooperation, Ulsan 44610 (KR)
(72) Inventor: KANG, Dong Wha, Seoul 05505 (KR); PARK, Inseok, Seoul 05505 (KR); CHOI, Hagyun, Seoul 05505 (KR); RYU, Yong Hoe, Seoul 05505 (KR)
(74) Representative: dompatent
(86) International application number: PCT/KR2024/007112
(87) International publication number: WO 2024/248430

(57) **Abstract**

Disclosed are a cognitive training providing apparatus and a method thereof. The cognitive training providing apparatus includes a display module, a memory that stores health status information of a trainee, pieces of multimedia content, and a plurality of training tasks, an input module that receives a response of the trainee, and a control module that selects first multimedia content corresponding to the health status information among the pieces of multimedia content, selects a first training task corresponding to the first multimedia content among the plurality of training tasks, organizes game content by placing the first training task in the first multimedia content, and performs a cognitive training operation by outputting the game content through the display module.

## Description

### [TECHNICAL FIELD]

Embodiments of the present disclosure described herein relate to a game content-based cognitive training providing apparatus and a method thereof, and more particularly, relate to a game content-based cognitive training providing apparatus for alternately providing overall brain stimulation and brain stimulation of specific areas, and a method thereof.

### [BACKGROUND ART]

Patients with partial brain damage due to diseases such as a stroke, dementia, and advanced age face many inconveniences in their daily lives. Cognitive training for improving a brain function has been recently used to treat these conditions, by using games such as virtual reality.

However, only simple repetition of conventional cognitive training was performed, which made the patients bored with the cognitive training or allowed the patients not to have immersed them in the cognitive training for a long time.

Accordingly, there is continuously required for an effective method to improve cognitive ability. The importance of an apparatus and a method for improving the cognitive ability by inducing a patient's immersion has increased.

### [DETAILED DESCRIPTION OF THE INVENTION]

### [TECHINICAL PROBLEM]

Embodiments of the present disclosure provide a game content-based cognitive training providing apparatus for alternately providing overall brain stimulation and brain stimulation of specific areas, and a method thereof.

Problems to be solved by the present disclosure are not limited to the problems mentioned above, and other problems not mentioned will be clearly understood by those skilled in the art from the following description.

### [TECHNICAL SOLUTION]

According to an embodiment, a cognitive training providing apparatus includes a display module, a memory that stores health status information of a trainee, pieces of multimedia content, and a plurality of training tasks, an input module that receives a response of the trainee, and a control module that selects first multimedia content corresponding to the health status information among the pieces of multimedia content, selects a first training task corresponding to the first multimedia content among the plurality of training tasks, organizes game content by placing the first training task in the first multimedia content, and performs a cognitive training operation by outputting the game content through the display module.

According to an embodiment, a cognitive training providing method performed by a control module of an apparatus includes obtaining health status information of a trainee, selecting first multimedia content corresponding to the health status information among pieces of multimedia content that are previously provided, selecting a first training task corresponding to the first multimedia content among a plurality of training tasks, organizing game content in which the first training task is placed in the first multimedia content, and performing a cognitive training operation by outputting the game content through a display module of the apparatus.

### [ADVANTAGEOUS EFFECTS OF THE INVENTION]

The cognitive training providing apparatus 100 according to an embodiment of the present disclosure may provide cognitive training by using game content composed of the plurality of episodes, thereby inducing the trainee to remain immersed while the trainee does not feel bored even after repeatedly performing cognitive training.

Moreover, the cognitive training providing apparatus 100 according to an embodiment of the present disclosure may induce the emotional immersion of the trainee by using game content having a story of a situation similar to a situation caused by the trainee's disease or symptom.

Furthermore, the cognitive training providing apparatus 100 according to an embodiment of the present disclosure may use the game content of cross-stimulation in which a training task providing brain stimulation of a specific area is placed between stories that provide overall brain stimulation of the trainee, thereby increasing the cognitive training effect of the trainee.

Besides, the cognitive training providing apparatus 100 according to an embodiment of the present disclosure may perform cognitive training appropriate to the status of the trainee by providing a training task with the adjusted difficulty.

### [DESCRIPTION OF THE DRAWINGS]

FIG. 1 is a block diagram of a cognitive training providing apparatus, according to an embodiment of the present disclosure.
FIG. 2 is a diagram illustrating a cognitive training providing method, according to an embodiment of the present disclosure.
FIG. 3 is a diagram for describing multimedia content, according to an embodiment of the present disclosure.
FIG. 4 is a diagram illustrating an overall brain stimulation training task, according to an embodiment of the present disclosure.
FIG. 5 is a diagram for describing a specific area brain stimulation training task, according to an embodiment of the present disclosure.
FIGS. 6 and 7 are diagrams for describing first and second implementation examples of a specific area brain stimulation training task, according to an embodiment of the present disclosure.
FIG. 8 is a flowchart for describing a method of organizing game content, according to an embodiment of the present disclosure.

### [BEST MODE]

The same reference numerals denote the same elements throughout the present disclosure. The present disclosure does not describe all elements of embodiments. Well-known content or redundant content in which embodiments are the same as one another will be omitted in a technical field to which the present disclosure belongs. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, an 'apparatus according to an embodiment of the present disclosure' includes all various devices capable of providing results to a user by performing arithmetic processing. For example, the apparatus according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

The server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet terminal (Wibro) terminal, and a wearable device such as a timepiece, a ring, a bracelet, an anklet, a necklace, glasses, a contact lens, or a head-mounted device (HMD).

Functions related to artificial intelligence according to an embodiment of the present disclosure are operated through a processor and a memory. The processor may consist of one or more processors. In this case, the one or more processors may be a general-purpose processor (e.g., a CPU, an AP, or a digital signal processor (DSP)), a graphics-dedicated processor (e.g., a GPU or a vision processing unit (VPU)), or an artificial intelligence (AI)-dedicated processor (e.g., an NPU). Under control of the one or more processors, input data may be processed depending on an AI model, or a predefined operating rule stored in the memory. Alternatively, when the one or more processors are AI-dedicated processors, the AI-dedicated processor may be designed with a hardware structure specialized for processing a specific AI model.

In this specification, an expression "task" may refer to a goal and/or objective to be achieved by a user. For example, the task may include a game or multimedia content. Moreover, a computerized task may be rendered by using computerized components, and a user may receive instructions regarding the goal or objective for performing the computerized task. The task may allow an individual to provide or withhold a response to a specific stimulus.

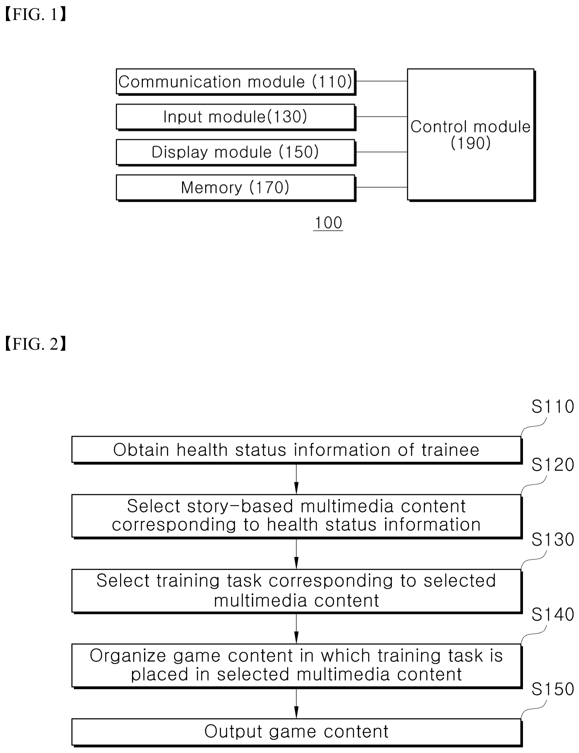

FIG. 1 is a block diagram of a cognitive training providing apparatus, according to an embodiment of the present disclosure. Referring to FIG. 1, a cognitive training providing apparatus 100 may include a communication module 110, an input module 130, a display module 150, a memory 170, and a control module 190. The components shown in FIG. 1 are not essential in implementing the cognitive training providing apparatus 100. The cognitive training providing apparatus 100 described herein may have more or fewer components than those listed above.

The cognitive training providing apparatus 100 according to an embodiment of the present disclosure may include various devices capable of performing calculation processing. For example, the cognitive training providing apparatus 100 may include a desktop PC, a mobile phone, a smart phone, a laptop computer, personal digital assistants (PDA), a portable multimedia player (PMP), a slate PC, a tablet PC, an ultra-book, a wearable device, and the like.

For example, the cognitive training providing apparatus 100 may include a head-mounted device such as a head mounted display (HMD) mounted on a trainee's head to display an image, smart glasses, or smart goggles, or a display device of a mobile phone capable of being mounted and used on the head-mounted device.

The communication module 110 performs wired or wireless communication with at least one external device (a server, etc.). In particular, when wireless communication is performed, wireless signals are exchanged over a communication network based on wireless Internet technologies.

For example, the wireless Internet technologies includes wireless LAN (WLAN), Wireless-Fidelity (Wi-Fi), Wi-Fi Direct, digital living network alliance (DLNA), wireless broadband (WiBro), world interoperability for microwave access (WiMAX), high speed downlink packet access (HSDPA), high speed uplink packet access (HSUPA), long term evolution (LTE), long term evolution-advanced (LTE-A), and the like. The cognitive training providing apparatus 100 transmits and receives data depending on at least one wireless Internet technology within a range including Internet technologies not listed above.

The communication module 110 may be used for short range communication, and may support short-range communication by using at least one of Bluetooth^{™}, radio frequency identification (RFID), infrared data association (IrDA), ultra wideband (UWB), ZigBee, near field communication (NFC), Wireless-Fidelity (Wi-Fi), Wi-Fi Direct, and wireless universal serial bus (Wireless USB) technologies. In this case, the short range wireless communication network may be wireless personal area networks.

The input module 130 may obtain a signal corresponding to a trainee's input. For example, the input module 130 may obtain the trainee's input for performing cognitive training, a response to a cognitive training operation provided through the display module 150, or the like.

In this case, the input module 130 may include a keyboard, a keypad, a button, a jog shuttle, a wheel, and the like. Besides, the trainee's input in the input module 130 may be, for example, pressing a button, touching, and dragging.

The input module 130 may be configured as a separate module connected to the cognitive training providing apparatus 100 in a wired or wireless manner. For example, the cognitive training providing apparatus 100 may provide a trainee with images for performing cognitive training through the display module 150 mounted on the trainee's head, and may receive a response from the trainee through the input module 130 implemented as a separate module given to the trainee's hand.

The display module 150 outputs videos or images. For example, the display module 150 may include an LCD, an OLED display, an AMOLED display, and the like. In an embodiment, when the display module 150 is implemented as a touch screen, the display module 150 may serve as the input module 130. In this case, the separate input module 130 may not be provided depending on a selection, or the input module 130 performing limited functions such as a volume control button, a power button, and a home button may be provided. Moreover, the display module 150 may be provided in a form of a video output port that transmits image information to an external display device.

In an embodiment, the display module 150 may include a first display and a second display, which respectively correspond to the trainee's eyes (a left eye and a right eye). Here, the first display may output a first image, and the second display may output a second image.

In the meantime, the present disclosure is not limited thereto, and the display module 150 may be implemented as a single display. In this case, the display module 150 may output the first image in a left area of the display and may output the second image in a right area of the display.

The memory 170 stores various pieces of data (information) as well as at least a piece of data (information) and at least one process, which are necessary to provide a cognitive training method. For example, a program for performing training, at least a piece of trainee information (personal information, health status information, response information, training results, or the like), a measurement image, a training image, or the like may be stored as data. Besides, the memory 170 may store various instructions, algorithms, or the like for performing a cognitive training operation.

Moreover, the memory 170 may include a storage medium of at least one type of a flash memory type, a hard disk type, a multimedia card micro type, a card type memory (e.g., SD memory, XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, an optical disc, etc. Besides, the memory 170 may store information temporarily, permanently, or semi-permanently, and may be provided in an embedded type or a removable type.

The control module 190 may control configurations within the cognitive training providing apparatus 100 or may process and calculate various pieces of information. Under control of the control module 190, the cognitive training operation may be performed based on at least one process stored in the memory 170.

The control module 190 may obtain health status information including information about the trainee's disease or symptom and may organize multimedia content corresponding to the trainee's disease or symptom, and game content including training tasks based on the obtained health status information. Furthermore, the control module 190 may perform a cognitive training operation by using the organized game content. In an embodiment, the multimedia content may be organized based on a story in which a player in a situation similar to a situation caused by a specific disease or symptom acts to achieve a specific goal. The multimedia content may induce emotional immersion of the trainee and may provide overall brain stimulation. Moreover, the training task may refer to cognitive training performed to improve a specific disease or symptom and may be configured to provide brain stimulation to specific areas of a trainee.

The control module 190 may be implemented with software, hardware, or a combination thereof. For example, in a hardware manner, the control module 190 may be implemented with a field programmable gate array (FPGA) or an application specific integrated circuit (ASIC), a semiconductor chip, or other various types of electronic circuits. Also, for example, in a software manner, the control module 190 may be implemented with various computer languages or a logic program executed depending on the above-described hardware.

Unless otherwise stated in the following description, it may be understood that an operation of the cognitive training providing apparatus 100 is performed under the control of the control module 190.

As such, the cognitive training providing apparatus 100 according to an embodiment of the present disclosure may induce the emotional immersion of the trainee by using game content having a story of a situation similar to a situation caused by the trainee's disease or symptom.

In the meantime, according to an embodiment, the cognitive training providing apparatus 100 may be implemented to perform a cognitive training operation through an external device. For example, when the cognitive training providing apparatus 100 is implemented as a server, the cognitive training providing apparatus 100 may perform the cognitive training operation by communicating with the external device through the communication module 110. In this case, an operation of using the input module 130 and the display module 150 of the cognitive training providing apparatus 100 described in this specification may be replaced with an operation of using a display module and an input module of the external device through the communication module 110.

FIG. 2 is a diagram illustrating a cognitive training providing method, according to an embodiment of the present disclosure. Referring to FIG. 2, the cognitive training providing apparatus 100 may obtain health status information of a trainee (S110). In detail, the cognitive training providing apparatus 100 may receive the health status information from an external device through the communication module 110 or may receive the trainee's health status information through the input module 130. When the health status information is stored in the memory 170, the cognitive training providing apparatus 100 may load the health status information from the memory 170. Here, the health status information may include at least one piece of information related to the trainee's health status, such as the trainee's disease, symptom, and age.

Moreover, the cognitive training providing apparatus 100 may select story-based multimedia content corresponding to the health status information (S120). In detail, the cognitive training providing apparatus 100 may select the multimedia content corresponding to the health status information among pieces of multimedia content including a story corresponding to a disease and/or a symptom. Each of the pieces of multimedia content may include pieces of image content and pieces of music content. Each of the pieces of multimedia content may include various pieces of content such as images, music, text, pictures, sound, and the like.

For example, the cognitive training providing apparatus 100 may select multimedia content of a story, in which a player, who has lost his/her memory due to an accident, takes various actions to achieve a given goal, in response to the health status information of the trainee with memory decline as a stroke patient. In other words, the cognitive training providing apparatus 100 may select multimedia content having a story similar to a situation caused by the trainee's disease or symptom such that the trainee may be emotionally immersed. For this reason, the multimedia content may have a warm-up effect of stimulating the trainee's brain overall and may maximize the effect of the training task.

Moreover, the cognitive training providing apparatus 100 may select a training task corresponding to the selected multimedia content (S130). Here, the training task may consist of cognitive training that stimulates a specific area of a brain corresponding to a specific disease or a specific symptom. For example, a training task corresponding to memory decline may consist of cognitive training that stimulates an area (e.g., a temporal lobe) of a brain that controls memory.

In detail, the cognitive training providing apparatus 100 may select a training task corresponding to the selected multimedia content based on matching information between multimedia content and a training task. For example, the matching information may be obtained by matching the multimedia content having a story corresponding to a stroke with the training task such as N-back tests, climbing stairs, finding directions, purchasing items, or the like. In other words, the matching information matches a training task capable of improving cognitive ability when the trainee with a disease and/or symptom corresponding to the multimedia content performs the training task. In an embodiment, the cognitive training providing apparatus 100 may select at least one or more training tasks.

In the meantime, the present disclosure is not limited thereto. According to an embodiment, the cognitive training providing apparatus 100 may be implemented to select a training task corresponding to the health status information. In another embodiment, the cognitive training providing apparatus 100 may be implemented to select a training task corresponding to the health status information and the selected multimedia content.

Besides, the cognitive training providing apparatus 100 may organize game content, in which a training task is placed in the selected multimedia content (S140). The cognitive training providing apparatus 100 may place at least one or more training tasks between pieces of multimedia content, thereby maintaining the trainee's immersion in the story of the multimedia content even in the training task. For example, the cognitive training providing apparatus 100 may organize game content such that a trainee should perform a training task in a process of story progression within the multimedia content and then may proceed to the next step.

Moreover, the cognitive training providing apparatus 100 may output the game content (S150). In particular, the cognitive training providing apparatus 100 may output the game content through the display module 150. Alternatively, according to an embodiment, the cognitive training providing apparatus 100 may transmit the game content to an external device through the communication module 110.

As such, the cognitive training providing apparatus 100 according to an embodiment of the present disclosure may use the game content of cross-stimulation in which a training task providing brain stimulation of a specific area is placed between stories that provide overall brain stimulation of the trainee, thereby increasing the cognitive training effect of the trainee.

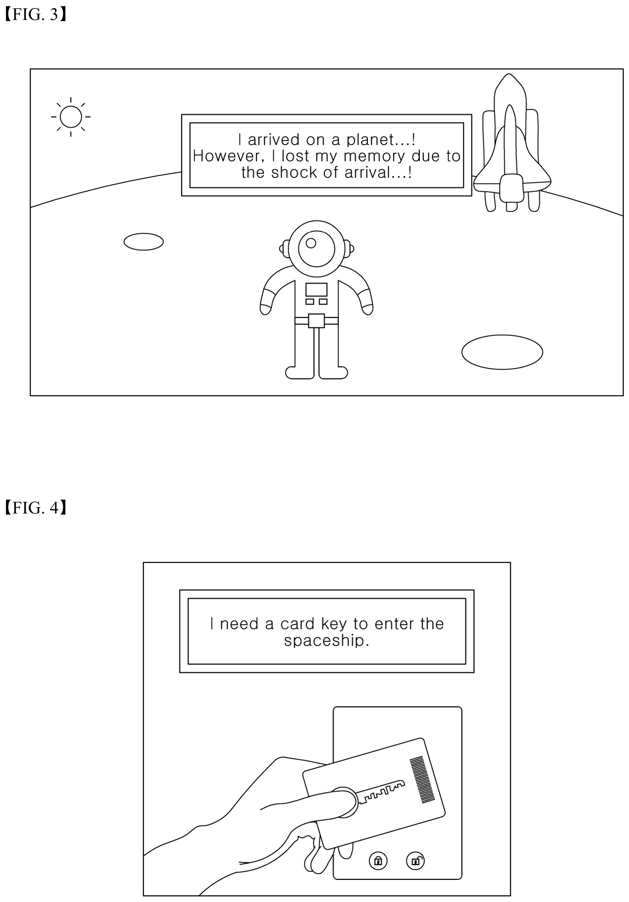

Hereinafter, detailed examples of game content will be described with reference to FIGS. 3 to 7. Moreover, for easier understanding, descriptions are given on the premise that the cognitive training providing apparatus 100 organizes game content corresponding to a stroke and memory decline.

FIG. 3 is a diagram for describing multimedia content, according to an embodiment of the present disclosure.

Referring to FIG. 3, the cognitive training providing apparatus 100 may select multimedia content, which is organized based on a story "a player who lost his/her memory after crashing on planet 'A' repairs a spaceship and escapes planet 'A'.", based on health status information including information about a stroke and memory decline. In other words, the cognitive training providing apparatus 100 may select multimedia content of the story such that a trainee with memory decline due to a stroke may be emotionally immersed in a situation in which a player lost his/her memory due to the crash. The multimedia content in FIG. 3 may include content such as various images, music, text, and pictures related to the story. The cognitive training providing apparatus 100 may output game content including the multimedia content to the display module 150. The trainee may perceive the multimedia content output through the display module 150 and may proceed with the story by entering a response through the input module 130.

FIG. 4 is a diagram illustrating an overall brain stimulation training task, according to an embodiment of the present disclosure.

In an embodiment, the cognitive training providing apparatus 100 may provide cognitive training of stimulating a specific area of a brain corresponding to a disease and/or a symptom, as well as cognitive training of stimulating overall areas of the brain. In other words, the training task may include a specific area brain stimulation training task of stimulating a specific area of the brain corresponding to the disease and/or the symptom, and an overall brain stimulation training task of stimulating the overall brain area. Here, the overall brain stimulation training task may be implemented as part of a warm-up task for maximizing the effect of the specific area brain stimulation training task rather than directly alleviating specific diseases or symptoms. In an embodiment, the cognitive training providing apparatus 100 may place not only the specific area brain stimulation training task but also the overall brain stimulation training task between pieces of multimedia content selected to organize game content.

For example, as an example of an overall brain stimulation training task, the cognitive training providing apparatus 100 may perform a task of picking up an object. Referring to FIG. 4, the cognitive training providing apparatus 100 may output multimedia content corresponding to "a situation in which a card key input is required to enter the spaceship" through the display module 150. Furthermore, the cognitive training providing apparatus 100 may request a trainee to perform the task of picking up an object while additionally outputting a card key image through the display module 150. The trainee may transmit a response (e.g., drag and drop) for moving the card key image to a door lock by using the input module 130 upon request. In addition, the cognitive training providing apparatus 100 may output the multimedia content corresponding to a story such that a player enters the interior of the spaceship depending on the trainee's response.

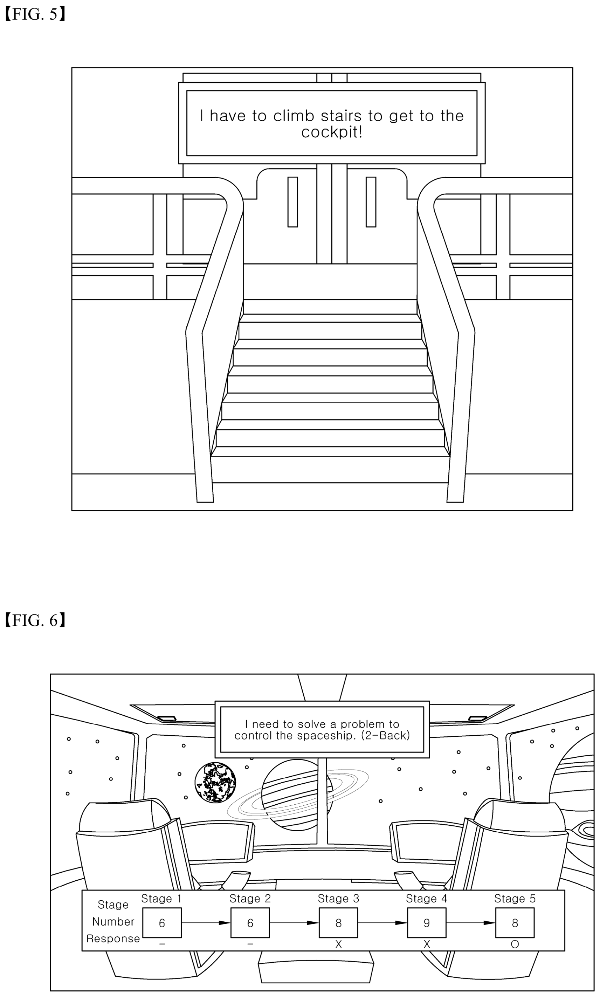

FIG. 5 is a diagram for describing a specific area brain stimulation training task, according to an embodiment of the present disclosure.

For example, as an example of a specific area brain stimulation training task corresponding to a stroke, the cognitive training providing apparatus 100 may proceed with climbing stairs. Referring to FIG. 5, the cognitive training providing apparatus 100 may output multimedia content corresponding to "a situation in which a player has to climb stairs to enter a cockpit in a spaceship" through the display module 150 and may request a trainee to perform a stair climbing task. The trainee may perform the stair climbing task upon request. For example, when the cognitive training providing apparatus 100 is implemented as a HMD, the trainee may perform the stair climbing task while wearing the cognitive training providing apparatus 100, and the cognitive training providing apparatus 100 may determine whether the stair climbing task is performed, based on the result of sensing an altimeter in the HMD. For another example, without separately sensing the altimeter, the cognitive training providing apparatus 100 may also determine whether the stair climbing task is performed, based on whether a response to completing the stair climbing task is received from the trainee or a user of the cognitive training providing apparatus 100. Moreover, the cognitive training providing apparatus 100 may output the multimedia content corresponding to a story such that the player enters the cockpit depending on the trainee's response.

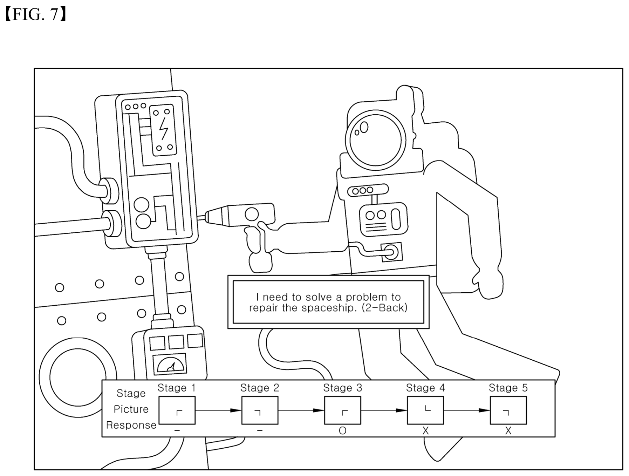

FIGS. 6 and 7 are diagrams for describing first and second implementation examples of a specific area brain stimulation training task, according to an embodiment of the present disclosure.

For example, as an example of a specific area brain stimulation training task corresponding to a stroke, the cognitive training providing apparatus 100 may perform N-back. The N-back refers to a test of determining whether N-th previous information matches current information. Referring to FIG. 6, the cognitive training providing apparatus 100 may output multimedia content corresponding to "a situation in which a problem needs to be solved to operate a spaceship" through the display module 150. Furthermore, the cognitive training providing apparatus 100 may request a trainee to perform 2-back task while additionally outputting 2-back task image through the display module 150. For example, referring to FIG. 6, the cognitive training providing apparatus 100 may sequentially display numbers 6, 6, 8, 9, and 8 for each stage and may output a task for determining whether the second previous number matches a current number starting from a third stage (stage 3). When the trainee succeeds in the 2-back task, the cognitive training providing apparatus 100 may output the multimedia content corresponding to a story in which the player controls a spaceship to escape a planet.

In the meantime, the cognitive training providing apparatus 100 may adjust the difficulty of game content according to an embodiment. The cognitive training providing apparatus 100 may adjust the difficulty of game content based on health status information or may adjust the difficulty of next game content based on previously performed training result information.

For example, both trainee A and trainee B are patients with a stroke, but trainee A may have a more severe stroke than trainee B. In this case, the cognitive training providing apparatus 100 may set the difficulty of game content provided to trainee B to be higher than the difficulty of game content provided to trainee A. For example, the cognitive training providing apparatus 100 may set the difficulty of the game content provided to trainee B in a method of increasing the difficulty of a training task or including a lot of training tasks. The changing of the difficulty of a training task may be made by changing details of the training task. Moreover, to reflect training tasks with increased difficulty or a lot of training tasks, the cognitive training providing apparatus 100 may also change or add multimedia content in response.

The cognitive training providing apparatus 100 may provide an n-back task with higher difficulty than the n-back task of FIG. 6. Referring to FIG. 7, the cognitive training providing apparatus 100 may output multimedia content corresponding to "a situation in which a problem needs to be solved to repair a spaceship" through the display module 150. Furthermore, the cognitive training providing apparatus 100 may request a trainee to perform 2-back task while additionally outputting 2-back task image through the display module 150. For example, referring to FIG. 7, the cognitive training providing apparatus 100 may sequentially display pictures '┌', '┐', '┌', '└', and '┐' for each stage and may output a task for determining whether the second previous picture matches a current picture starting from a third stage (stage 3). When the trainee succeeds in the 2-back task, the cognitive training providing apparatus 100 may output the multimedia content corresponding to a story in which the player repairs a spaceship to escape a planet.

In the meantime, the method of adjusting the difficulty of an n-back task is not limited to determining the sameness by using numbers or pictures. For example, the method may be implemented in various ways, such as increasing the number of n or limiting the trainee's response time.

As such, the cognitive training providing apparatus 100 according to an embodiment of the present disclosure may provide cognitive training appropriate to the status of the trainee by providing a training task with the adjusted difficulty.

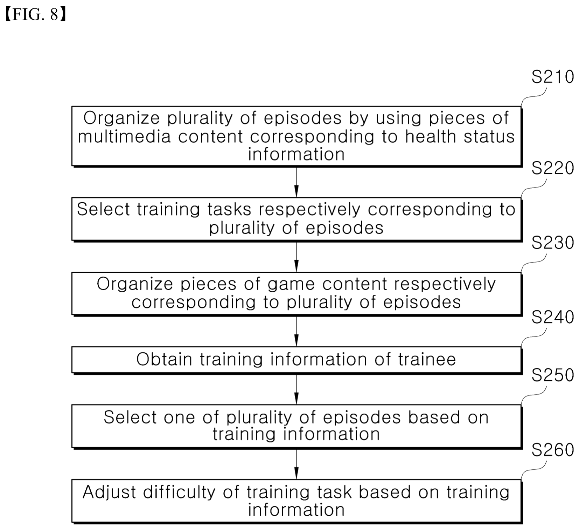

FIG. 8 is a flowchart for describing a method of organizing game content, according to an embodiment of the present disclosure. In detail, FIG. 8 is a flowchart for describing a method of organizing game content composed of a plurality of episodes.

Referring to FIG. 8, the cognitive training providing apparatus 100 may organize a plurality of episodes by using pieces of multimedia content corresponding to health status information (S210). In detail, the cognitive training providing apparatus 100 may select pieces of multimedia content corresponding to the health status information among the pieces of multimedia content including a story corresponding to a disease and/or a symptom, Moreover, the cognitive training providing apparatus 100 may organize the pieces of multimedia content, which are selected, into episodes, each of which is the same as or smaller than a unit. For example, with regard to the multimedia content described above in FIGS. 3 to 7, the cognitive training providing apparatus 100 may organize all the pieces of multimedia content into one episode, or may divide all the pieces of multimedia content into two or more stories to organize a plurality of episodes.

In an embodiment, each of the plurality of episodes may be composed of individual stories. In the meantime, the present disclosure is not limited thereto. For example, each of the plurality of episodes may be composed of stories that are connected to each other, or only some of them may be composed of individual stories.

Furthermore, the cognitive training providing apparatus 100 may select training tasks respectively corresponding to the plurality of episodes (S220). In detail, the cognitive training providing apparatus 100 may select at least one or more training tasks respectively corresponding to the plurality of episodes. For example, in a state where the cognitive training providing apparatus 100 organizes a first episode and a second episode, types of training tasks may include a first training task, a second training task, and a third training task. Besides, the cognitive training providing apparatus 100 may select the first training task with respect to the first episode and may select the first training task and the second training task with respect to the second episode. In addition, the third training task may be excluded from the selection because the third training task does not correspond to diseases and/or symptoms of the first episode and the second episode.

Also, the cognitive training providing apparatus 100 may adjust details of a training task selected to correspond to each of a plurality of episodes. In the above example, even when the first training task is selected with respect to both the first episode and the second episode, details of the first training task may be adjusted to suit each episode. For example, when the first episode includes solving problems with numbers, the cognitive training providing apparatus 100 may adjust the first training task to an n-back task using numbers. Moreover, when the second episode includes solving problems with pictures, the cognitive training providing apparatus 100 may adjust the first training task to an n-back task using pictures. In other words, in addition to adjusting the details of the training task to adjust the difficulty described above in FIGS. 6 and 7, the cognitive training providing apparatus 100 may adjust the details of the training task for each episode.

Accordingly, even when repeatedly performing a specific brain area stimulation training task, a trainee may not feel that he/she is repeating the same task, thereby preventing boredom or loss of immersion.

Furthermore, the cognitive training providing apparatus 100 may organize pieces of game content respectively corresponding to the plurality of episodes (S230). In detail, the cognitive training providing apparatus 100 may organize game content such that the corresponding training task is placed between pieces of multimedia content corresponding to the corresponding episode with respect to each of the plurality of episodes.

Besides, the cognitive training providing apparatus 100 may obtain training information of the trainee (S240). Here, the training information may be information about previously performed training and may include multimedia content information, training task information, episode information, training scores, and the like. In an embodiment, the training information may be stored in the memory 170. In the meantime, when the training information is not present (e.g., when cognitive training is performed for the first time), operation S240 may be omitted.

In addition, the cognitive training providing apparatus 100 may select one of the plurality of episodes based on the training information (S250). In detail, the cognitive training providing apparatus 100 may identify previous episode information included in the training information and may select one of the remaining episodes excluding the previously performed episode.

When the training information is not present, the cognitive training providing apparatus 100 may select one from all episodes. In this case, the method of selecting an episode may be set to randomly select an episode or to select a specific episode first by default.

Moreover, the cognitive training providing apparatus 100 may adjust the difficulty of the training task based on the training information (S260). In detail, the cognitive training providing apparatus 100 may identify the previous training score included in the training information and may adjust the difficulty of the training task so as to correspond to the previous training score. For example, when the previous training score is lower than a reference value, the cognitive training providing apparatus 100 may decrease the difficulty of the training task. Furthermore, when the previous training score is higher than the reference value, the cognitive training providing apparatus 100 may increase the difficulty of the training task. In the meantime, the method of adjusting the difficulty of the training task is not limited to the above-described example. For example, the cognitive training providing apparatus 100 may be implemented to split training results into a plurality of ranges and to adjust the difficulty of the training task to the difficulty corresponding to each range.

When the training information is not present, the difficulty of the training task may be set to the default value. Alternatively, according to an embodiment, the cognitive training providing apparatus 100 may adjust the difficulty of the training task based on health status information.

The cognitive training providing apparatus 100 may provide the trainee with game content composed through the above-described operations. In particular, the cognitive training providing apparatus 100 may output the game content through the display module 150. Alternatively, according to an embodiment, the cognitive training providing apparatus 100 may transmit the game content to an external device through the communication module 110.

Meanwhile, the disclosed embodiments may be implemented in a form of a recording medium storing instructions executable by a computer. The instructions may be stored in a form of program codes, and, when executed by a processor, generate a program module to perform operations of the disclosed embodiments. The recording medium may be implemented as a computer-readable recording medium.

The computer-readable recording medium may include all kinds of recording media in which instructions capable of being decoded by a computer are stored. For example, there may be read only memory (ROM), random access memory (RAM), magnetic tape, magnetic disk, flash memory, optical data storage device, and the like.

Disclosed embodiments are described above with reference to the accompanying drawings. One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

## Claims

1. A cognitive training providing apparatus, the apparatus comprising:
a display module;
a memory configured to store health status information of a trainee, pieces of multimedia content, and a plurality of training tasks;
an input module configured to receive a response of the trainee; and
a control module configured to:
select first multimedia content corresponding to the health status information among the pieces of multimedia content;
select a first training task corresponding to the first multimedia content among the plurality of training tasks;
organize game content by placing the first training task in the first multimedia content; and
perform a cognitive training operation by outputting the game content through the display module.

2. The apparatus of claim 1, wherein the health status information includes at least one of a disease, a symptoms, and an age of the trainee, and
wherein the control module is configured to:
select the first multimedia content including a story corresponding to the disease and/or the symptom of the trainee based on the health status information.

3. The apparatus of claim 1, wherein the plurality of training tasks include a specific area brain stimulation training task of stimulating a specific area of a brain corresponding to a specific disease or a specific symptom.

4. The apparatus of claim 1, wherein the control module is configured to:
select the first training task corresponding to the first multimedia content by using matching information obtained by matching the pieces of multimedia content with the plurality of training tasks based on a disease and/or a symptom.

5. The apparatus of claim 1, wherein the plurality of training tasks include an overall brain stimulation training task of stimulating overall areas of a brain.

6. The apparatus of claim 1, wherein the control module is configured to:
adjust difficulty of at least one of the plurality of training tasks.

7. The apparatus of claim 1, wherein the control module is configured to:
adjust difficulty of next cognitive training based on a result of previous cognitive training of the trainee.

8. A cognitive training providing method performed by a control module of an apparatus, the method comprising:
obtaining health status information of a trainee;
selecting first multimedia content corresponding to the health status information among a pieces of multimedia content that are previously provided;
selecting a first training task corresponding to the first multimedia content among a plurality of training tasks;
organizing game content in which the first training task is placed in the first multimedia content; and
performing a cognitive training operation by outputting the game content through a display module of the apparatus.

9. The method of claim 8, wherein the health status information includes at least one of a disease, a symptoms, and an age of the trainee, and
wherein the selecting of the first multimedia content includes:
selecting the first multimedia content including a story corresponding to the disease and/or the symptom of the trainee based on the health status information.

10. The method of claim 8, wherein the plurality of training tasks include a specific area brain stimulation training task of stimulating a specific area of a brain corresponding to a specific disease or a specific symptom.

11. The method of claim 8, wherein the selecting of the first training task includes:
selecting the first training task corresponding to the first multimedia content by using matching information obtained by matching the pieces of multimedia content with the plurality of training tasks based on a disease and/or a symptom.

12. The method of claim 8, wherein the plurality of training tasks include an overall brain stimulation training task of stimulating overall areas of a brain.

13. The method of claim 8, further comprising:
adjusting difficulty of at least one of the plurality of training tasks.

14. The method of claim 8, further comprising:
adjusting difficulty of next cognitive training based on a result of previous cognitive training of the trainee.

15. A computer-readable recording medium storing a computer program for performing the cognitive training providing method of claim 8.
